# EUROPEAN PATENT SPECIFICATION

(11) **EP 1 196 082 B1**
(45) Date of publication and mention of the grant of the patent: **10.03.2010**
(21) Application number: 00944933.1
(22) Date of filing: 28.06.2000
(51) Int. Cl.: A61B 5/00, A61N 1/372

(54) **SYSTEM FOR REMOTE COMMUNICATION WITH AN IMPLANTABLE MEDICAL DEVICE**
SYSTEM ZUR FERNKOMMUNIKATION MIT EINEM IMPLANTIERBAREN MEDIZINISCHEN GERÄT
SYSTEME DE COMMUNICATION A DISTANCE AVEC UN DISPOSITIF MEDICAL IMPLANTABLE

(30) Priority: 07.07.1999 US 348506
(43) Date of publication of application: 17.04.2002
(73) Proprietor: Medtronic, Inc., Minneapolis, MN 55432-5604 (US)
(72) Inventor: FEREK-PETRIC, Bozidar, 10000 Zagreb (HR)
(74) Representative: Hughes, Andrea Michelle
(86) International application number: PCT/US2000/017699
(87) International publication number: WO 2001/003575

(56) References cited:
- WO-A-98/24358
- WO-A-98/42407
- WO-A-99/18532

## Description

The present invention relates to medical devices and, particularly, to a system for the remote communication with a medical device.

Medical devices are currently implanted in a variety of patients for a variety of reasons. Such medical devices may include, without limitation, drug pumps, nerve stimulators, cardiac defibrillators, as well as cardiac pacemakers. Once implanted, many of these medical devices require the download of data collected by the medical device, the reprogramming of the medical device or the interrogation of the medical device to ensure proper operation, among others.

Subsequent communication with an already implanted medical device generally requires the use of a programmer. Programmers are devices which permit the downlink to be made from the programmer to the medical device as well as the receipt of a subsequent uplink from the medical device to the programmer. Often the programmer is placed within the hospital or physician's office so that the physician is directly next to the patient while the communication with the medical device is made. Thus, present devices often require the patient to travel directly to the hospital or doctor's office to permit the communication to be made with the medical device.

Such travel, merely for the communication with the medical device, may sometimes be a problem. Patients may live in a variety of locations, including such remote locations as islands, mountainous areas or other areas of the world where travel is difficult as well as remote from the particular physician's office, Moreover, on occasion the attending physician may desire additional input or guidance for the data received from the medical device and the subsequent reprogramming.

WO-98/42 407 shows a system according to the preamble of claim 1.

In view of the above, there exists the need for a system that permits remote communication with a medical device. There exists a further need for such remote communication to be permissible such that one or more device experts such as physicians and more experienced device users may be aware of the communication and provide guidance for the subsequent interpretation and programming of the device.

Accordingly, the present invention provides a system for remote communication with a medical device according to claim 1.

Preferred embodiments are set out in the dependent claims.

The system particularly may permit the remote communication such that one or more device experts such as physicians and more experienced device users may be aware of the communication and provide guidance for the subsequent interpretation and programming of the device.

Preferred embodiments will now be described, by way of example only, with reference to the drawings.
FIGs. 1A & 1B depicts an overview of a system according to the present invention.
FIGs. 2A & 2B depict further embodiments of a system according to the present invention.
FIGs. 3A & 3B are schematic block diagrams of steps undertaken for the communication with a medical device using a system of the present invention.
FIG. 4 depicts a preferred safety feature of the present invention.
FIG. 5 depicts steps used to permit the authorized level client usage in the programmer.
FIG. 6 depicts the various types of information transferred between client, server and device as well as a protocol used for the transmission.
FIG. 7 shows a system operation for checking information received along the second data channel.
FIG. 8 shows basic steps used between a client, server and programmer to transmit a command.
FIG. 9 depicts an alternative embodiment.
FIG. 10 depicts the steps used to permit a variety of different clients to be logged in to a single server.
FIG. 11 illustrates a system substantially like that shown in FIG. 1A but also featuring a time & autocorrelator 111 communicating with a signal receiver 112 and signal generator 113 in client.
FIG. 12 depicts an alternative embodiment.

The FIGS. are not necessarily to scale.

The present invention relates to a system for remote communication with a medical device. This system particularly takes advantage of the Internet.

The Internet, sometimes called simply "the Net," is a worldwide system of computer networks - a network of networks in which users at any one computer can, if they have permission, get information from any other computer (and sometimes talk directly to users at other computers). It was conceived by the Advanced Research Projects Agency (ARPA) of the U.S. government in 1969 and was first known as the ARPANet. The original aim was to create a network that would allow users of a research computer at one university to be able to "talk to" research computers at other universities. A side benefit of ARPANet's design was that, because messages could be routed or rerouted in more than one direction, the network could continue to function even if parts of it were destroyed in the event of a military attack or other disaster.

Today, the Internet is a public, cooperative, and self-sustaining facility accessible to hundreds of millions of people worldwide. Physically, the Internet uses a portion of the total resources of the currently existing public telecommunication networks. Technically, what distinguishes the Internet is its use of a set of protocols called TCP/IP (Transmission Control Protocol/Internet Protocol). Two recent adaptations of Internet technology, the intranet and the extranet, also make use of the TCP/IP protocol.

The Internet Protocol (IP) is a protocol or method by which data is sent from one computer to another on the Internet. Each computer (known as a host) on the Internet has at least one address that uniquely identifies it from all other computers on the Internet. When you send or receive data (for example, an e-mail note or a Web page), the message gets divided into little chunks called packets. Each of these packets contains both the sender's Internet address and the receiver's Internet address. Any packet is sent first to a gateway computer (router) that understands a small part of the Internet. The gateway computer reads the destination address and forwards the packet to an adjacent gateway that in turn reads the destination address and so forth across the Internet until one gateway recognizes the packet as belonging to a computer within its immediate neighborhood or domain. That gateway then forwards the packet directly to the computer whose address is specified.

Because a message is divided into a number of packets, each packet can, if necessary, be sent by a different route across the Internet. Packets can arrive in a different order than the order they were sent in. The Internet Protocol just delivers them. It's up to another protocol, the Transmission Control Protocol (TCP) to put them back in the right order.

IP is a connectionless protocol, which means that there is no established connection between the end points that are communicating. Each packet that travels through the Internet is treated as an independent unit of data without any relation to any other unit of data. (The reason the packets do get put in the right order is because of TCP, the connection-oriented protocol that keeps track of the packet sequence in a message.) In the Open Systems Interconnection (OSI) communication model, IP is in layer 3, the Networking Layer.

The most widely used version of IP today is Internet Protocol Version 4 (IPv4). However, IP Version 6 (IPv6) is also beginning to be supported. IPv6 provides for much longer addresses and therefore for the possibility of many more Internet users. IPv6 includes the capabilities of IPv4 and any server that can support IPv6 packets can also support IPv4 packets.

UDP (User Datagram Protocol) is a communications method or protocol for communication which offers a limited amount of service when messages are exchanged between computers in a network that uses the Internet Protocol (IP). UDP is an alternative to the Transmission Control Protocol (TCP) and, together with IP, is sometimes referred to as UDP/IP. Like the Transmission Control Protocol, UDP uses the Internet Protocol to actually get a data unit (called a datagram) from one computer to another. Unlike TCP, however, UDP does not provide the service of dividing a message into packets (datagrams) and reassembling it at the other end. Specifically, UDP doesn't provide sequencing of the packets that the data arrives in. This means that the application program that uses UDP must be able to make sure that the entire message has arrived and is in the right order or that the mis-ordering or loss of data is hot of consequence. Network applications that want to save processing time because they have very small data units to exchange (and therefore very little message reassembling to do) may prefer UDP to TCP. The Trivial File Transfer Protocol (TFTP) uses UDP instead of TCP.

UDP provides two services not provided by the IP layer. It provides port numbers to help distinguish different user requests and, optionally, a checksum capability to verify that the data arrived intact.

Computers that are on the Internet utilizing TCP/IP protocol are usually so called open systems continuously connected within the local area network infrastructure. Hubs are used for these mutual connections, while specialized computers, routers or gateways, are used for linking LANs over the long distances. Computers may be continuously connected to the leased line dedicated for Internet traffic or they may be connected on-demand via modems and telephone lines. However, there are many computers which temporarily become a part of the Internet utilizing the dial-up connection. This kind of Internet connection is usually done from home. The low-level protocols used to carry Internet traffic over a dial-up connection are called SLIP (Serial Line Internet Protocol) and PPP (Point-to-Point Protocol). In comparison with the full-time connection, this kind of connection is slower. Nevertheless, ISDN (Integrated Services Digital Network) becomes available even at homes, offering higher speeds than the standard modem connection. While the connection speed between the two IP addresses is much higher through the dedicated full-time connection, the connection speed may be sloped down due to them intensive traffic on the network wherein many users send and receive the data. Routers and hubs usually treat the users to have equal priority and distribute the traffic throughout the network in order to achieve the best throughput rate utilizing the free network resources. Therefore sometimes intermittent interruption of the data transmission and reception may occur and the average throughput rate may be very low on the busy network.

Accordingly, the throughput rate between the two computers may be faster and more reliable utilizing dial-up connection. This is because of the fact that hubs and routers are avoided utilizing direct telephone line connection by means of the modem or the ISDN interface. In dial-up connection, the telephone line is reserved only for the TCP/IP traffic between the two computers.

At present, the most convenient computer platform and language for implementation of this invention is Java^{™} which may be provided by Sun Microsystems, Inc., San Jose, Calif. Java is a high-level object-oriented interpreted programming language being architecture-neutral and portable. Java has a compiler for translating a Java program into an intermediate language called Java bytecodes, the platform-independent codes interpreted by the Java interpreter. With an interpreter, each Java bytecode instruction is parsed and run on the computer. Compilation happens just once; interpretation occurs each time the program is executed. Java bytecodes are like the machine code instructions for the Java Virtual Machine (Java VM). Every Java interpreter, whether it's a Java development tool or a Web browser that can run Java applets, is an implementation of the Java VM. The Java VM can also be implemented in hardware. The Java platform has two components: the Java Virtual Machine (Java VM) and the Java Application Programming Interface (Java API). The Java API is a large collection of ready-made software components that provide many useful capabilities, such as graphical user interface (GUI) widgets. The Java API is grouped into libraries (packages) of related components. Probably the most well-known Java programs are Java applets. An applet is a Java program that adheres to certain conventions that allow it to run within a Java-enabled browser. Common type of Java programs are also applications, where a Java application is a standalone program that runs directly on the Java platform. A special kind of application known as a server, serves and supports clients on a network. Other specialized programs are servlets which are similar to applets in that they are runtime extensions of applications. Instead of working in browsers, though, servlets run within Java servers, configuring or tailoring the server. The huge variety of implementations of this invention, which may be done utilizing Java platform and language, prevents the precise disclosure of all the aspects. The following figures are only examples for illustration, but an expert skilled in the art may use Java in numerous different possible modes because it is among the most dynamic, simple and multithreaded computer platform and language.

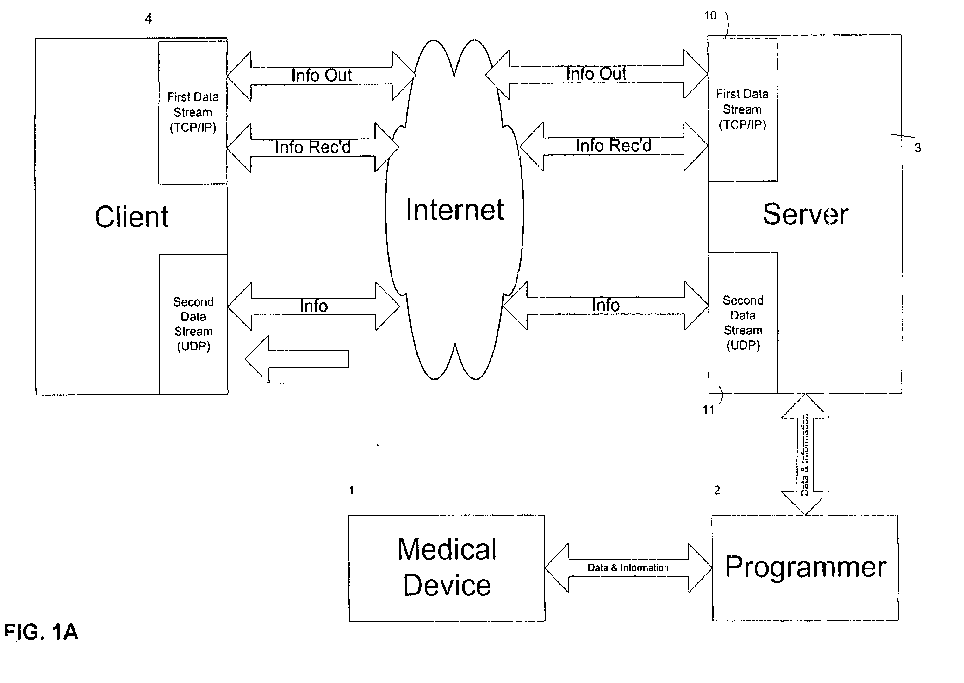

FIG. 1A depicts an overview of one system according to the present invention. Due to the fact that the system should operate within the computer network, it may be, but is not necessarily, built as a client/server configuration. The Internet is based upon data which may be offered to client computers which can hook-up to the various kinds of servers - ftp, wais, web etc. A typical client is the PC comprising web browser software. Since the occurrence of Java platform, the possibility to share the complexity of the specific software task is particularly attractive by exchange of the applets. Moreover, special server application and special client application open the possibility that the hardware and processing resources are shared in such a way as to optimize the tasks for the particular implantable device programming session.

As seen, the system has essentially five primary components. Medical device 1 may be of any type which provides therapy to a patient or information collection so long as the device has the ability for communication (unlink and downlink) to an external device, seen here as programmer 2. Medical device may, for example only and without limitation, comprise a Medtronic Kappa^{™} 400 DDDR pacemaker. Programmer 2 may, again, be of any acceptable design which permits linkage to a medical device including the Medtronic 9790 programmer.

The server is a computer which permits the transfer of information, including data and commands, from device 1, programmer 2 and server 3 to client 4 through a dispersed network, depicted here as Internet 5. Network traffic flows between client and server through two separate data streams 10 and 11.

First data stream 10 transmits information which has been divided into discrete information packets wherein control messages and state updates between the server and the client are comprised. In the preferred embodiment, first data stream 10 transmits information using the TCP/IP network protocol containing both the sender's Internet address as well as the receiver's address. If a dial-up connection is used, one of the low-level protocols are used (either SLIP or PPP). Regardless of the specific protocol used, the first data stream is designed so that information (such as data or commands) which is sent from one computer to the other has a receipt which is sent in the opposite direction so that the transmission of the information is verified. Such a data stream transmission is reliable but has a relatively slow rate or data transfer. It warrants either delivery or failure notification, therefore being used for data such as commands to the server and messages to clients designating the server changes. It may also be used to send the calculated heart beat rate to the clients after each beat. As this message is sent periodically and rather often, it may serve as a periodical probe to measure the connection's reliability. As the Internet protocols will evolve, the system may be adapted utilizing the more efficient protocol such as the Internet Protocol Version 6.

The second data stream transmits information using a different protocol. As seen, such a protocol has the transmission in only one direction, that is, this data channel does not transmit information having the returned receipt request, as required in the first data stream. For this reason the second data stream is relatively faster than the first data stream, although has a lower degree of reliability since the actual receipt of the information cannot always be verified.

In the preferred embodiment, the first data stream transmits commands and other types of information while the second data stream transmits the real-time ECG waveform of the medical device. In the preferred embodiment, the second data stream transmits data using User Datagram Protocol ("UDP"). As mentioned above, unlike TCP, the UDP does not provide the service of dividing the message into packets and reassembling it at the other end of the transmission. Specifically, UDP does not provide sequence to the packets when the data arrives. This means the programmer that uses UDP must make sure that the entire message has arrived and is in the right order or that missing or mis-ordered data is of no consequence.

In the present invention, the second data stream is used for transmission of the ECG, hence, the UDP data consists of only two bytes per message - one data point of the ECG waveform and its ordinal number. Apart from the ECG waveform; the cardiac therapy device could also send the IEGM, some hemodynamic parameter waveform such as blood pressure, blood flow, or even the ultrasound measurement waveform. UDP can be also used for transmission of the marker channels as well as of the various biochemical sensors signals. For example, within the context where the medical device is a nerve stimulator (similar to the Medtronic Itrel^{™} series) the device could transmit an EEG or EGG waveform. In fact, a nerve stimulator having the capability to measure the tremor intensity would transmit the signal representing the tremor intensity thus enabling the DBS output programming according to the tremor intensity. Since digitized waveforms have an inherent redundancy, in that the general waves are known as well as the possible values of the different parameters, missing data packets or missing data may be quickly identified as being actually missing, as opposed to indicating very low sensed and reported signal strength, or the data having peculiar values. UDP packets are satisfactory for this purpose because it is not crucially important that every packet arrives. Missing data packets, however, should not normally happen on a dedicated network link and therefore this event may be used as an additional test of the network link. That is the amount of missing data packets may be used as a parameter to indicate to the users the fidelity of the link. Such fidelity may be communicated through a pop-up menu or box on one or more of the users screens. This box may indicate that the network transmissions are not complete and that data is missing. Courses of action may thereafter be recommended, including restarting the system, or reissuing the command. It must be understood, however, that other protocols for data transfer may also be used, other than only UDP.

In a further embodiment regarding the data link, the delay between the data at the server and its receipt at the client may also be provided to one or more of the users. The delay display is of value to the users as it permits the basis for the lag between commands to be understood as possibly only a network phenomena. Delay display may be provided in many acceptable manners, including that of the UNIX command "traceroute."

Here is an example of the traceroute command execution for the connection between the two computers in Zagreb, Croatia (maja.zesoi.fer.hr) and up to the ftp server of Medtronic Inc. The measurement stopped at some security firewall of the Medtronic network which didn't allow further penetration. Delays to establish connection through various gateways, together with the relevant IP addresses, are revealed. traceroute to ftp.medtronic.com (144.15.255.25), 30 hops max, 40 byte packets
1 zemsgwy (161.53.64.1) 2 ms 2 ms 1 ms
2 161.53.113.65 (161.53.113.65) 4 ms 1 ms 1 ms
3 194.152.222.97 (194.152.222.97) 5 ms 6 ms 4 ms
4 bordercore30-hssi0-1-0.Westorange.cw.net (166.49.27.9) 445 ms 432 ms *
5 xcore.Chicago.cw.net (204.70.150.1) 380 ms 377 ms 344 ms
6 mci-gw.mixnet.net (204.70.186.6) 375 ms 412 ms 468 ms
7 Border0-F5-0.MSC.MR.Net (198.174.96.2) 376 ms 351 ms 412 ms
8 * Border35-e0.MSC.MR.Net (137.192.3.35) 472 ms 381 ms
9 Medtronic-1-MRNet.MSC.MR.Net (137.192.27.150) 359 ms 378 ms 400 ms
10 * * *

Accordingly, the result of the data packets delivery time, revealed by the traceroute command, may be directly utilized to evaluate the feasibility of the connection for the teleprogramming procedure.

In a further embodiment the system may feature a communication link test capability. Through this capability, the system may determine whether it is in fact the communication link which is faulty (e.g. poor network quality) or whether it is instead a programming wand communication problem. This communication link test capability would essentially operate as follows: First a test waveform would be generated by the client and transmitted through the network thereby received by the server.

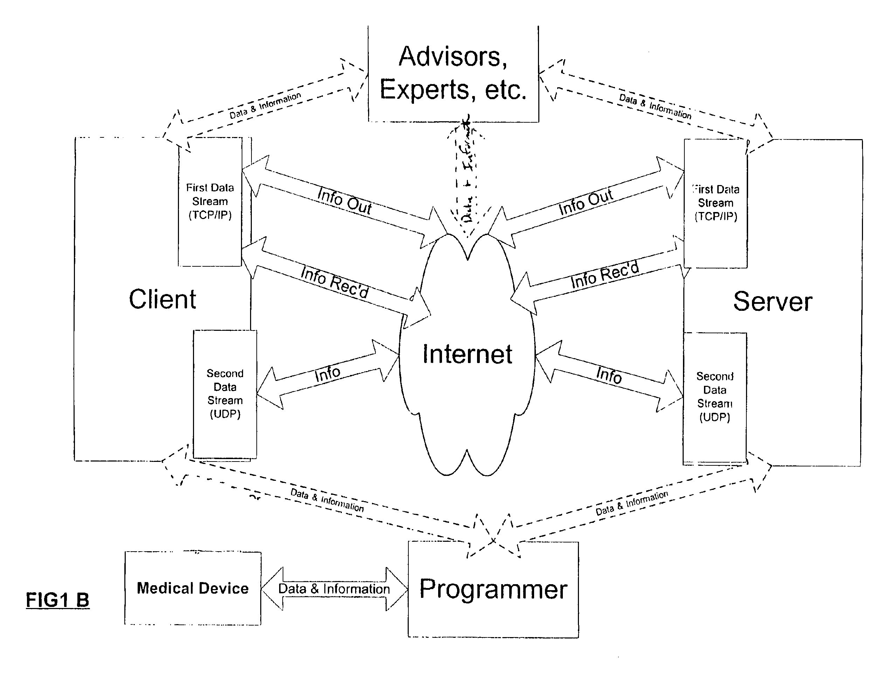

FIG. 1B depicts an overview of an alternative system according to the present invention. As seen in this embodiment the system is the same as that already described in FIG. 1A but for the additional provision of one or more advisors or experts, each of whom may communicate through the server, client or the Internet.

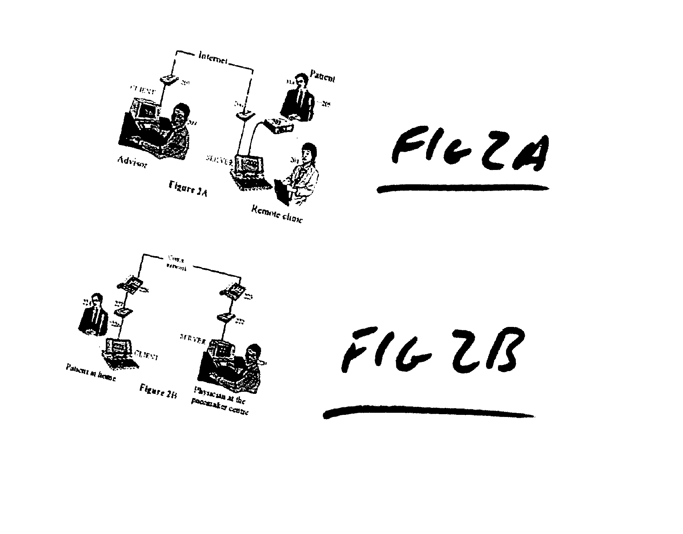

FIG. 2A illustrates further details of the possible system embodiment. The server 200 is installed in the remote clinic operated by the technician 201. In this embodiment, programmer 203 having an ECG interface is a separate device and is connected to server 200. Patient 204 is connected by the ECG cable (not shown) to the interface 203 for the purpose of the ECG recording, while programming wand 205 through an electromagnetic coupling enables, as is well know in the medical device field, the communication with the implanted device (not shown). The server 200 is the most complex component of the system, which provides real-time data about the patient's state, accepts and executes the operator's commands, monitors the system's overall performance and reliability, resolves all dubious situations and acts in emergency situations. The server is designed to take all this responsibility because it is nearest to the patient, and failure of any of the components between it and the patient are least likely. It may be a.standard PC computer, but also a "black box" without special screen and command buttons which could confuse the operator 201. A variety of different types of devices may be used to connect server 200 to the Internet (which usually comprises hubs and routers which are not shown) including modem, ISDN or ATM interface 206. A second device is provided to connect client 208 to the Internet. A modem 207 is illustrated as connecting client 208 to the Internet (of course other devices such as ISDN or ATM interface may also be used.) Advisor 209, being the expert for the particular therapy with the medical device, is the operator of the client computer 208. The client 208 shows to the operator 209 a graphic user interface that visually and functionally mimics the GUI of the implantable device programmer 203. It displays the ECG waveform of the patient, and comprises the controls of the ECG recording and display as well as the implantable device programming commands.

FIG. 2B discloses another possible embodiment of the system. In particular this illustrates a system used to provide pacemaker patient remote follow-up. As seen, pacemaker expert 220 is in the pacemaker center operating the server computer 221. Server 221 is connected to the normal telephone line appliance 223 by means of the modem 222. Patient 224 operates himself a device 225 being both the client computer and the pacemaker programmer. Therefore it comprises a programming wand 226 for communication with the implantable device (not shown). It may also comprise some kind of simple patient ECG cable (not shown) such as metal bracelets. Modem 227 is used to connect the client 225 to the normal telephone line appliance 228. In this configuration, the server shows the GUI of the pacemaker programmer including the patient real time ECG waveform. The patient connects the ECG bracelets (not shown) on his arms and positions the programming wand over the implanted device. Client computer 225 runs previously installed device specific software application. Patient can initiate the call to the appropriate dial-in network number and logging into the server. Software application of the server 221 recognizes the client 225 and displays the client specific GUI on the screen of the server. The voice over data feature may be possible, for the purpose of the communication between the physician 220 and the patient 224. Physician 220 can interrogate and program the implanted device while monitoring the ECG waveform of the patient 224. In this embodiment, the client computer and the programmer should be integrated into the one low-cost simple device for easy application. Though client 225 is illustrated as a notebook computer, it could be designed as a simple "black box" without the screen comprising only the basic command buttons and LED signals easily operated by average patient. The disclosed principle of the client at the patient side and the server at the advisory side, may also be used in the previously disclosed Internet connection system.

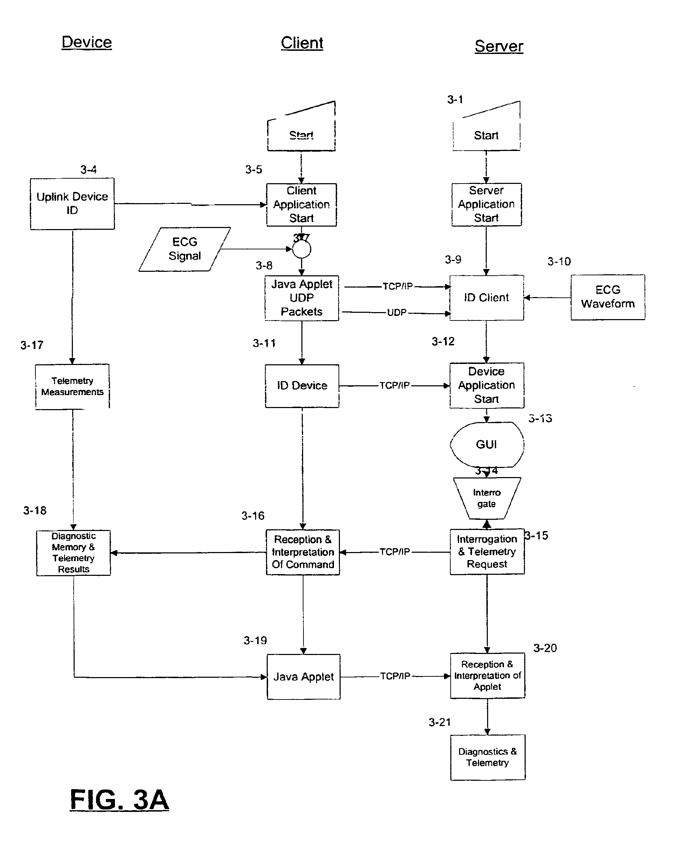

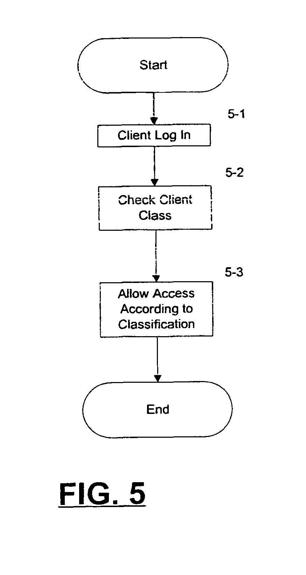

FIG. 3A is a schematic block diagram of steps undertaken for the communication with a medical device using the system of the present invention disclosed in FIG. 2A. As seen at 3-1, the server is logged into. Thereafter, communication with the programmer is begun 3-2, whereby the server takes the control over the programmer. Thereafter, at 3-3 the programmer identifies the medical device through uplinking in identify step, step 3-4, whereby the device sends also the present status, such as those provided by the QuickLook^{™} feature of the Vision^{™} software, the product of Medtronic, Inc. At 3-5 the server starts the software application for the specific device, preferably written in Java language. At any time during the abovementioned period the client login takes place at 3-6 including the process of the user classification 3-7 (illustrated below in FIG. 5) and the client software application start preferably written in Java language. At 3-8 client sends the appropriate request to the server in order to establish the mutual communication whereby client gets the previously defined assignment according to the user class either as an advisor who can take over the follow-up control or only as an observer. As discussed above, this takes place via the Internet utilizing one of the various communication protocols, including TCP/IP. At 3-9 the server identifies the client user and, thereafter, authorization is provided to the client at 3-10 from the server including recognition of the user class and definition of the corresponding Java applet This is an important feature of the system as it permits users having differing access levels to be provided the appropriate GUI. Programmer provides the server with the ECG waveform of the patient at 3-11. Such information is provided from the medical device to the programmer in a well known manner.. The server prepares the UDP packets comprising the information about the ECG waveform and sends them to the client at 3-12 as well as the Java applet for the GUI on the client. Applet is received and interpreted within the client Java application at 3-13 and appropriate GUI is displayed together with the real time ECG waveform of the patient wherein authorized advisor has the graphic icons and buttons for the programmer control. At 3-14 a device interrogate command is given. This may be accomplished, for example, by pointing to an "Interrogate" button within the client GUI, the server receives the command at 3-15 and the programmer requests interrogation from the implanted device at 3-16. Upon the telemetry measurements at 3-17, the device may be providing telemetry output, seen at 3-18, to the programmer. As seen, telemetry received by the programmer at 3-19 is further provided to the server which prepares the appropriate Java applet at 3-20 providing the applet to the client via the network. Client interprets the Java applet by its Java application software at 3-21 and displays the telemetry results and diagnostic memory content within its GUI.

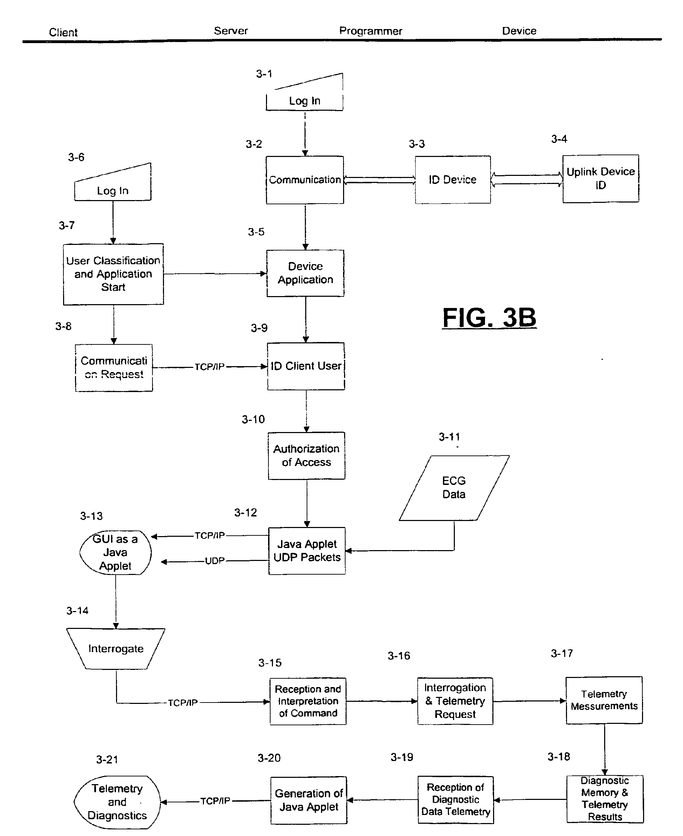

FIG. 3B is a schematic block diagram of the steps undertaken for the communication with a medical device using the system of the present invention disclosed in FIG. 2B. It is assumed for this illustration that the programmer and the client are a single hardware device. Of course, this is not per se required and separate and discrete devices may also be used. As seen at 3-1, the server is logged into. Thereafter, the server software application is begun at 3-2, whereby server waits for dial-up network connection call. At any time, at 3-3 the patient activates his device and the client application starts at 3-4. Patient puts the programming wand above the implanted device and the client identifies the medical device through uplinking in identified step, step 3-5. Patient connects the ECG cable to his body starting to record the ECG waveform at 3-6 supplying the client with the ECG signal at 3-7. At 3-8 client makes a call sending the appropriate request to the server in order to establish the dial-up network connection whereby starting to send a real-time ECG waveform in UDP packets as well as the patient data. As discussed above, this takes place via the Internet using various communication protocols, including TCP/IP. The server identifies the client at 3-9 and the real-time ECG waveform is continuously displayed at 3-10 within the server GUI together with the all of the patient data. Client sends the device identification at 3-11 and server starts the software application for the specific device at 3-12. This causes the change of the GUI at 3-13 bringing display of the additional menus and buttons for interrogation and programming of the specific device. After uplink, the device has automatically completed the telemetry measurements at 3-17. Operator of the server points to the appropriate button at 3-14 putting an interrogation and telemetry request at 3-15 to the client through the network which receives and interprets this command at 3-16. As seen, diagnostic memory content and telemetry results are prepared at 3-18 for the processing in order to make a Java applet at 3-19 and to send it via network. The server receives and interprets the applet at 3-20 and displays the telemetry and diagnostics screen at 3-21. This may be the screen such as the QuickLook^{™} feature of the vision^{™} software, the product of Medtronic, Inc.

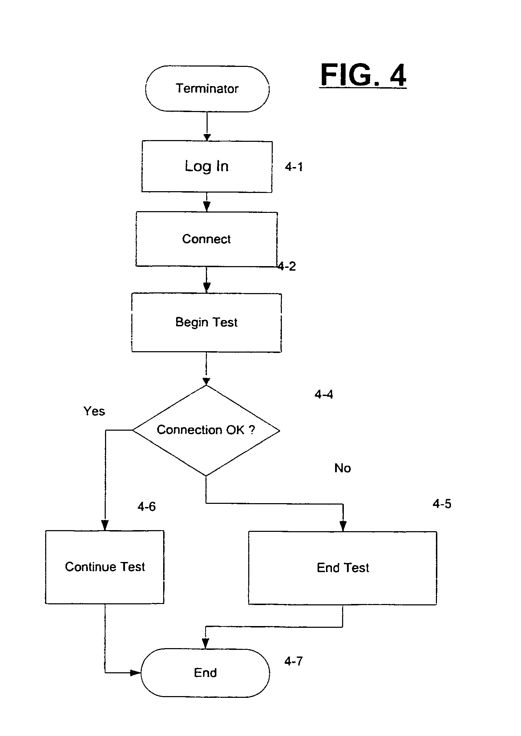

FIG. 4 depicts a safety feature of the present invention. As can be appreciated, during remote communication, the remote communication link may be, on occasion, interrupted. Medical devices often, during such remote communication, are called on to perform various tests. Such tests, although generally safe, should not be unavoidably interrupted or, further, should not be permitted to continue should an interruption occur. The steps used in FIG. 4 provide a method by which a test which has begun in the device may be stopped should the remote connection be interrupted. As seen at 4-1, the client is logged in and a connection made at 4-2. At some time, thereafter, a test is begun in the device, 4-3. While the device testing is begun the server maintains a watch to maintain if the connection is okay, 4-4. As seen, if the connection is not okay, the device drops down and ends the test immediately, 4-5. If the connection is okay, the device drops to 4-6 where the test is continued. Thereafter, the device continues with the test before recycling again back to block 4-4, where the connection is again verified to be okay. Subsequently, upon test end, the device would drop through block 4-7 and the test would be ended.

FIG. 5 depicts the steps used to permit the authorized level client usage in the programmer. As seen at 5-1, the client logs in. Subsequent to this, at 5-2. the server checks the client classification and user access. The server can have this information stored either in the server memory or, more preferably, itself access the third party database to permit identification of the proper client access. Thereafter, at 5-3 the server allows the logged in client access according to the classification of the client. Thereafter, the programming and device communication may continue as desired.

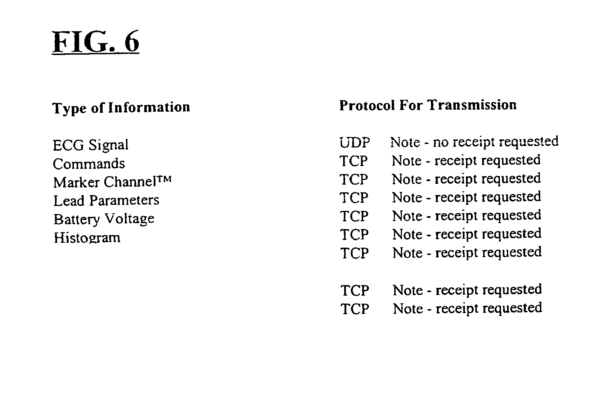

FIG. 6 depicts the various types of information transferred between client, server and device as well as the protocol used for the transmission. As discussed above, the system uses at least two communication channels, the first channel requiring a receipt returned. The second data channel, in contrast, does not provide a receipt that the information was properly received. As seen, the system preferably uses a non-receipt information protocol for information such as the QRS signals of a pacemaker. Such information is ongoing display information in which the absence of data may be readily ascertained as well as not immediately crucial to device operation. Other information, such as commands, are transmitted using a protocol in which a receipt of acceptance is provided.

In the preferred embodiment, the first channel protocol is UDP, as mentioned above, while the second data channel is a TCP, also mentioned above. Other information transmitted using such TCP includes, in the example of a pacemaker, marker channel, battery voltage levels, histogram information as well as lead parameters.

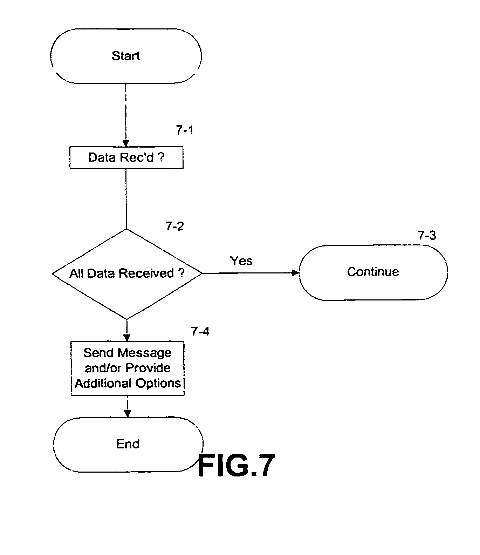

FIG. 7 shows a system operation for checking information received along the second data channel. As discussed above, the second data channel does not provide back to the sender a receipt. The steps used in FIG. 7 permit the sender to be notified by the recipient if an incorrect or improper amount of information is received by the receiver. As seen at step 7-1, the receiver determines whether any information has been received. At 7-2 the receiver starts the dedicated signal processing for the purpose of the waveform analysis in order to determine whether all the data has been received at 7-3. Determination that all the data has been received may be provided manually, by the operator, or automatically, by resident software. Such resident software may be any of the various types of signal processing, including morphologic filtering, pattern recognition, templates, etc. If yes, then the receiver continues to step 7-4, otherwise the receiver drops down to 7-5. At 7-5 the receiver sends a message to the sender corresponding to the type of data that is missing. In the example of a cardiac pacemaker, the information which may be sent in such a manner is the QRS signal. In this example where the QRS signal is received but some of the data seems to be missing according to the client, the client channels a message at 7-4 which is sent to the server and, thus, to the programmer that the ECG connection should be inspected.

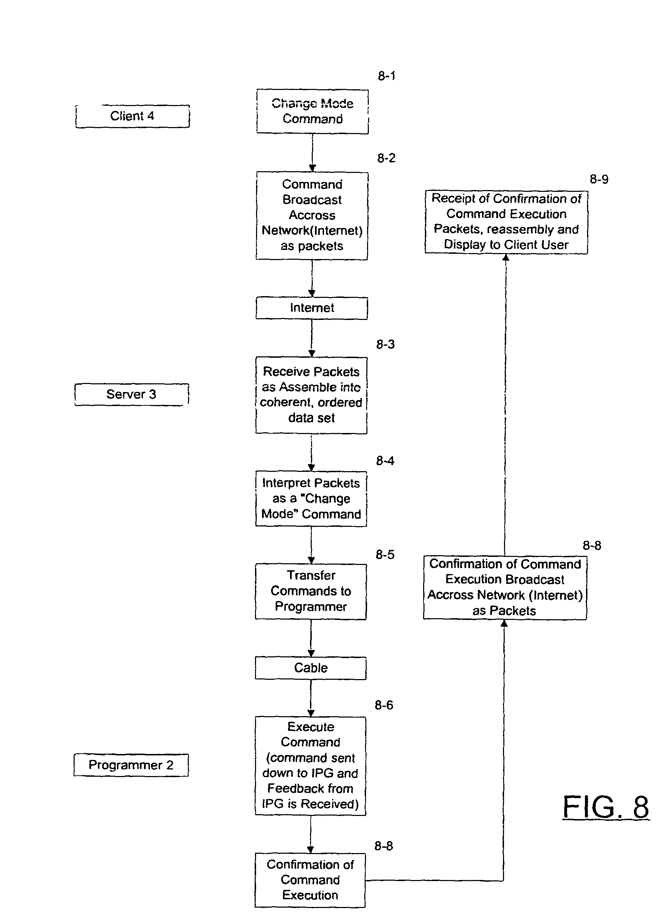

FIG. 8 shows basic steps used between a client, server and programmer to transmit a command. As seen at 8-1, a change mode command is entered by the user into the client. At 8-2 the change mode command is broadcast across the network or internet as a series of packets using an appropriate internet protocol, TCP in the preferred embodiment. At 8-3 server receives the packets and assembles them into the proper order. At this time the server would also send back to the client a receipt that the packet sent off at 8-2 has been received. At 8-4 the server interprets the received packets which have now been assembled as a change mode command. At 8-5 the change mode command is transferred to the programmer. As seen, commands are transferred to the programmer via a cable. At 8-6 the programmer executes a command by down linkage to the IPG which does change its mode, and which responds that the mode has been changed back to the programmer. At 8-7 a confirmation of command execution is sent back to the server again via the cable. At 8-8 the confirmation of command execution is broadcast across the network as packets by the server. At 8-9 the client receives the packets broadcast by the server and assembles them, thereafter, also interpreting and displaying the packets as a change mode command executed.

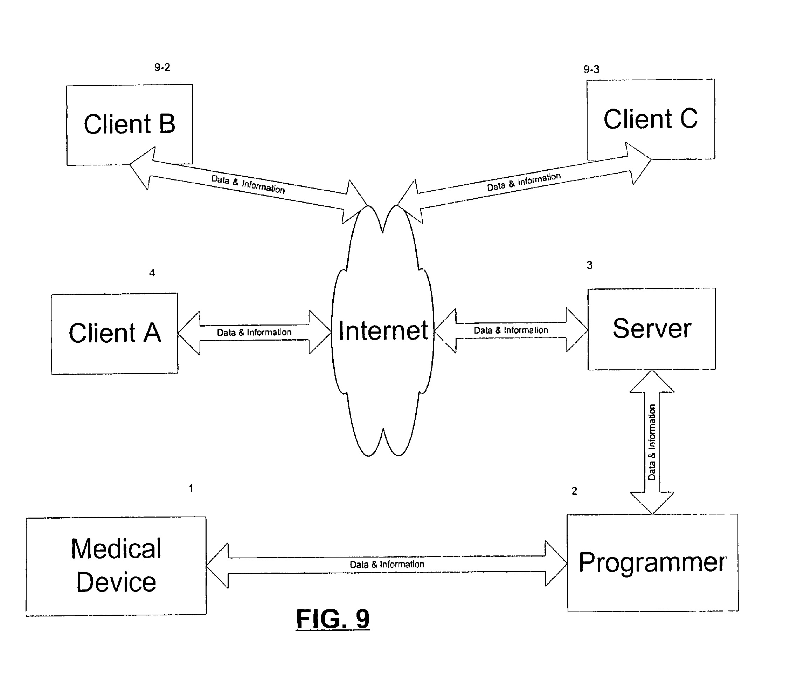

FIG. 9 depicts an additional system to the present system. The present system permits communication to be made over dispersed mediums such as an internet. As such it permits ready access by a multiple number of clients to a single server. Thus, as seen, the system could permit, in addition to client A, depicted here as 4 (and which is communicated with and to in the same manner as that discussed with regards to FIG. 1), the additional provision of client B 9-2 and client C 9-3. These additional clients may have varying means of access to the server and, thus, to the programmability of the device. For example, client B may have access to be only an observer while client C may be provided to be an observer/client with the additional ability to interrogate the device as well as do memory and telemetry read out. In this environment, moreover, server 3 plays a more active role. For example, as discussed above each client may have various level of access. To permit this, server 3 provides differing GUI applets to the differing authorized clients. For example, only one authorized client would have the possibility to control the programming session i.e. program the therapy device and to see the private patient data. Other clients would only have the possibility to observe what the authorized client is doing. They could also save the interrogated program and telemetry data as well as the ECG, IEGM and marker channel waveforms in their computers for the later recall for educational purpose.

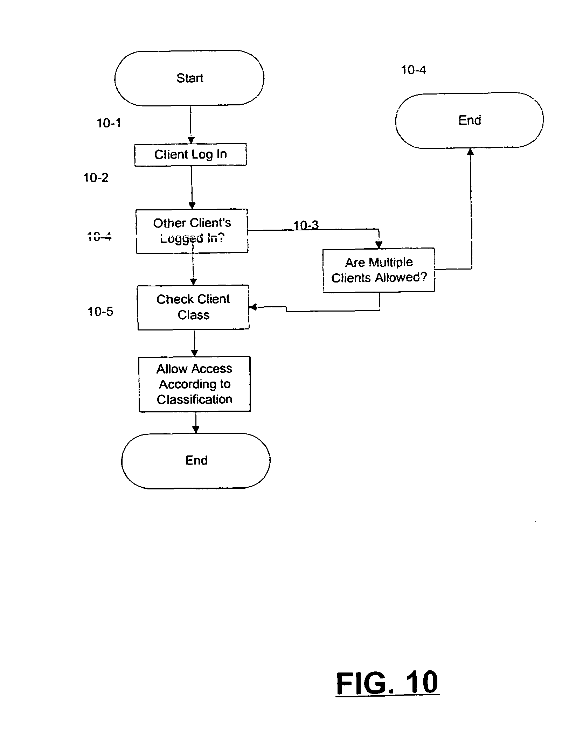

FIG. 10 depicts the steps used to permit a variety of different clients to be logged in to a single server. Thus, FIG. 10 depicts the steps used to achieve a system such as that shown in FIG. 9. At 10-1 the client logs in to the server. At 10-2 the server determines whether other clients are already logged in. If yes, then the server proceeds to 10-3 and determines whether multiple clients are permitted to be logged in. If not, then the client attempting to log in at 10-1 is denied access and the process ends, depicted here as 10-4. If, however, multiple clients may be logged in 10-3, then the system proceeds to 10-4 where the client classification is checked. Thereafter, the device proceeds to 10-5 and allows the client access according to their classification. Thereafter, the process may continue where the client receives the information or commands the device, or both, as already discussed above.

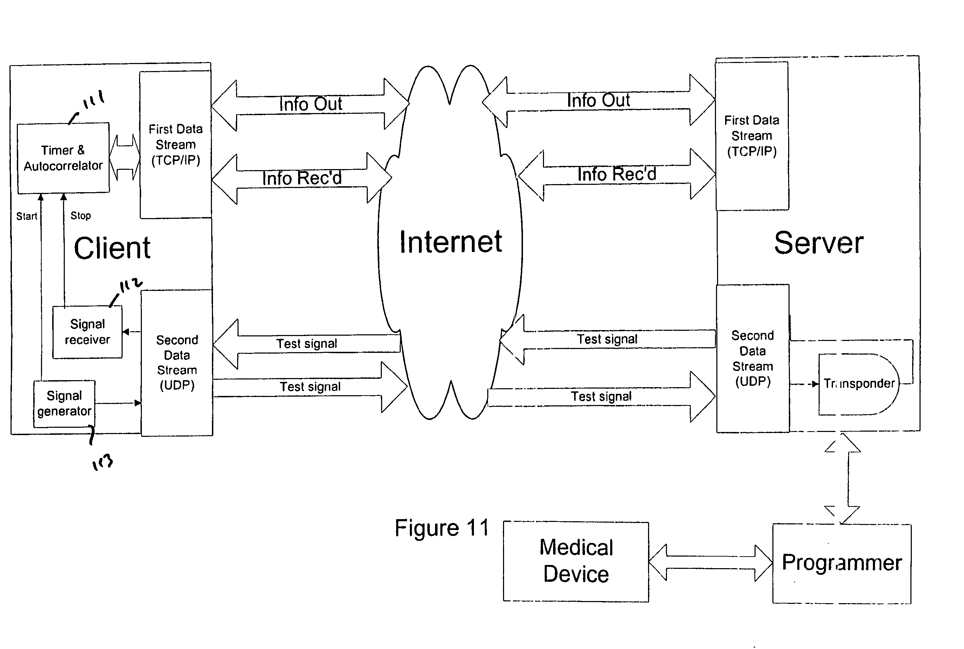

FIG. 11 illustrates a system substantially like that shown in FIG. 1A but also featuring a time & autocorrelator 111 communicating with a signal receiver 112 and signal generator 113 in the client. Also shown is a transponder 114 in server. These components may be used to provide a system which will permit a test signal routine to be enabled so as to determine and thereafter indicate the delay or lag in the signal. Either one of the client or server, or the programmer, for that matter, may thereafter be equipped to indicate to one or more system operators or advisor as to the speed or lag of the signal over the network One of the methods to exactly measure the signal delay is to use the RF signal of the atomic clock emitting exact coded time. For the two places, very far from the clock, the code is received with negligible difference in time and appropriate software could be used for reading the time of transmission as well as the time of reception. While the traceroute UNIX command would be feasible for the majority of applications, particularly for the continuously defined connections, it would never be able to evaluate the connection quality i.e. degradation of the signal's waveform through the network transfer. The simple method would be the "software transponder" such as disclosed in figure 11. Transponder is normally a device which sends instantly upon the signal's reception the signal back to the transmitter of that particular signal. It also may involve some delay which depends usually on electronic circuits. Nevertheless, it is usually used for the certain remote identification of the remote receiver. Despite the fact that it is usually the high frequency hardware, it can be implemented as a software within the computer. In the case of the remote programming technology, the special software could be built for evaluation of the various delays generated throughout the network. The transponder may be incorporated either in the client or in the server. The illustrated example discloses the client comprising the test signal generator and the test signal receiver as well as the timer and the autocorrelator. The server comprises the transponder. Apart from the disclosed example it may be implemented vice versa with the transponder in the client and signal generator, receiver and timer-autocorrelator in the server. The client initiates the test signal generation sending it as UDP to the client via Internet infrastructure, giving the start reference time at the same moment to the timer-autocorrelator. The test signal's waveform is provided to the autocorrelator. After the reception in the server, the signal is bounced back immediately as UDP via Internet with the known measurable delay of the transponding process. Upon the signal reception in the client, the stop time is issued to the timer-autocorrelator. The time between the transmission and the reception of the test signal is equal to the network delay of the UDP channel. Moreover, the autocorrelation between the transmitted and the received waveform may be done in order to evaluate the eventual degradation of the signal quality by means of the network transfer. Time measurement, autocorrelation, transmission, reception and transponding are very well known processes in software engineering and the expert skilled in the art could implement. A still further embodiment will now be discussed. The network infrastructure continues to become more and more advanced through the introduction of the new technologies. In 1993, the Asynchronous Transfer Mode (ATM) has been introduced to enable the very high data throughput rate for the high reliability and high speed connections consolidating data, audio and video traffic over a single broad-bandwidth line. It offers the cost-effective multiple services over the same network whereby ATM transmission equipment is easily configured to adapt variable traffic patterns and priorities for various tasks: LAN interconnect, frame relay, high-speed data services and IP routing.

ATM brings completely new possibilities for telemedicine and particularly for the implantable devices follow-up. IP over ATM will be the most important variant of the infrastructure utilized for the public network. ATM is a connection-oriented technology utilizing the Layer 2 cell switching of the Open Systems Interconnection protocol that is hardware based. In contrast to that, IP is a connectionless protocol whereby IP networks use software-based Layer 3 packet routing. From the practical point of view, the most convenient for teleprogramming would be the ATM connection between the client and the server, regardless which one of the two comprises the programmer. It may happen that commercial interest will rise among the medical institutions and small physicians offices in such a manner that they will have multiple tasks and therefore cheap ATM connection for teleradiology, telepathology, teleconference, telesurgery and other various telemedicine applications. Nevertheless, several IP over ATM protocols are in development. Particularly the Internet Engineering Task Force (IETF) has been active developing the RFC 1577 being the first true IP over ATM technology called Classical IP (CLIP) and emulating the IP subnetwork in an ATM infrastructure. One or more logical internetworking subnetworks are connected to a backbone subnetwork comprising routers attached to an ATM node. ATM Forum has developed LAN emulation (LANE) providing the protocols used by the clients to dynamically setup and tear-down ATM data carrying connections over the backbone network. It has a lack of security standards for the public networks. Ipsilon Networks developed the IP switching being actually a combination of intelligent IP routing with high-speed ATM switching, having major lack of scalability. ATM Forum developed a novel approach to the overlay combining the LANE dynamic setup process with the another routing protocol called Next Hop Resolution Protocol (NHRP). Finally, Multiprotocol Label Switching (MPLS) is the emerging technology of the IETF utilizing the concepts of the IBM's label switching in ARIS protocol and of the TAG switching of Cisco.

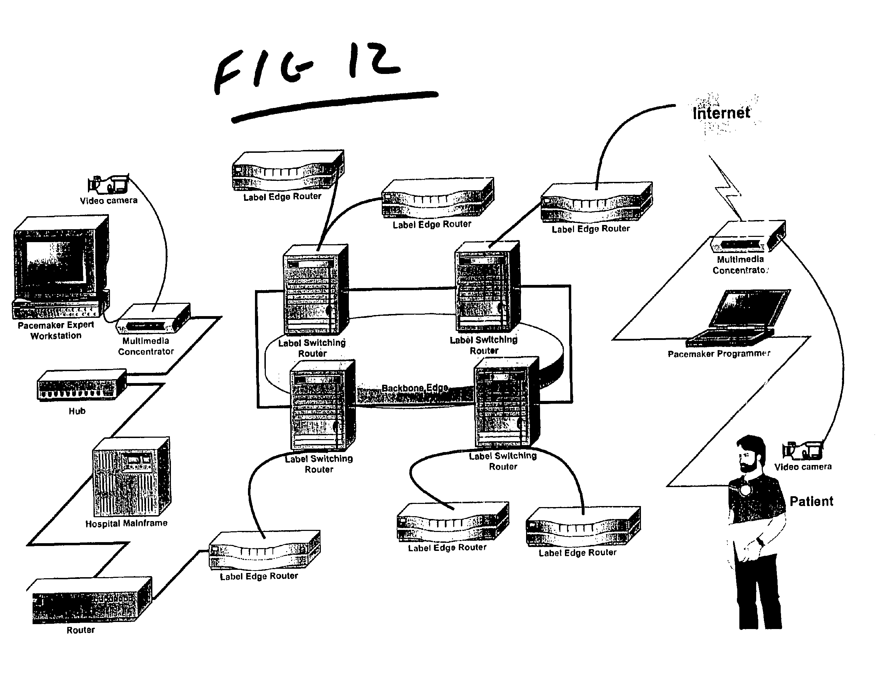

FIG. 12 illustrates an implantable device teleprogramming system utilizing the MPLS protocol in the future modern public network infrastructure based upon the ATM. The pacemaker expert workstation and the pacemaker programmer have the capability of data, audio signal, and video signal transmission and reception. Accordingly, they would practically integrate the technology of the teleconference and the technology of the teleprogramming. That also requires the capability of audio-video compression and decompression (MPEG-2). A multimedia network access concentrator may be used in order to concentrate the video and data traffic onto one network interface. Intra-domain routing protocols identify a predetermined path between any two points. A label is associated with the specific path and appended to the IP packet by a Label Edge Router. The packet is forwarded to a Label Switch Router (LSR) wherein the label is read and the packet is forwarded to its destination. Dynamic establishment and removal of label switched paths is controlled by the Label Distribution Protocol which allows transparency of the topology information between the nodes. The forwarding function is done within the ATM switch because MPLS eliminates the need for a router subnetwork structure utilizing the LSR as an ATM switch. The result is the IP switching at the speed of the ATM backbone switching enabling the real time multi-channel exchange of the biomedical signals, transmission of the high-resolution medical images and finally the audio-visual contact between the expert advisor and the remote patient site. Though the programming wand is disclosed, it will not be necessary in the future. Telemetry circuits are in the process of development which will enable the connection between the patient and the programme within the range of everyday room activities. Accordingly, the physician will be able to login into the patients device and check its function. The physician would be automatically alarmed about the occurrence of the hazardous arrhythmia.

Thus there is disclosed a system permitting the remote communication with a medical device. The system particularly may permit the remote communication such that one or more device experts such as physicians and more experienced device users may be aware of the communication and provide guidance for the subsequent interpretation and programming of the device.

## Claims

1. A system for remote communication with a medical device comprising
a medical device adapted to be implanted into a patient;
a server PC (221) communicating with the medical device, the server PC (SPC) having means for transmitting data across a dispersed data communication pathway; and
a client PC (225) having means for receiving data transmitted across a dispersed communication pathway from the server PC, the server PC having means for transmitting data across a dispersed data communication pathway along a first channel (10) and a second channel (11),
the client PC having means for receiving data transmitted across a dispersed communication pathway from the server PC along said first channel and said second channel,
**characterised by** the first channel (10) using a first data protocol requiring a confirmation of receipt to be transmitted back along said first channel, and the second channel (11) using a second data protocol not requiring a confirmation of receipt and providing transmission of data only in one direction.

2. The system according to claim 1 further comprising a programmer (2), the programmer coupled to the server PC and providing a means for interacting with a medical device.

3. The system according to claim 2 wherein the means for interacting with an medical device comprise a means for testing the medical device.

4. The system according to claim 2 or 3 wherein the programmer (4) has means for sensing a connection interruption between the client PC and server PC or the server PC and programmer .

5. The system according to claim 4 wherein the programmer (2) has means for terminating any testing being performed by the medical device should the means for sensing the connection interruption sense a connection interruption.

6. The system according to claim 2 wherein the means for interacting with an medical device comprise means for interrogating the medical device

7. The system according to claim 2 wherein the means for interacting with an medical device comprise means for downloading data from the medical device

8. The system according to claim 2 wherein the means for interacting with an medical device comprise means for uploading data to the medical device

9. The system according to any preceding claim wherein the client PC has means for communicating across a dispersed data communication pathway to the server PC.

10. The system according to any preceding claim wherein the client PC has means for classifying client users as either a first client user or a second client user.

11. The system according to any of claims 1 to 9 wherein the client PC has means for classifying client users as either a first client user or a second client user or a third client user or a fourth client user.

12. The system according to claim 10 or 11 further comprising means for providing a first user interface to the first client user upon the classification of the client user as a first client user.

13. The system according to claim 12 further comprising means for providing a second user interface to the client user upon the classification of the client user as a second client user.

14. The system according to claim 10, 11, 12 or 13 further comprising means for limiting access to the medical device upon the classification of the client user as a first client user.

15. The system according to claim 14 wherein the mean for limiting access to the medical device upon the classification of the client user as a first client user comprises means for permitting the first client user to only observe medical device operation.

16. The system according to clam 15, wherein the medical device has means for storing information, and wherein the means for limiting access to the medical device upon the classification of the client user as a first client user comprises mean for permitting the first client user to only retrieve information stored in the medical device.

17. The system according to any preceding claim wherein the means for transmitting data across a dispersed data communication pathway comprises means for transmitting data in one or more data packets.

18. The system according to claim 17 wherein the one or more data packets each have a confirmation receipt whereby the client PC, upon receipt of each of the one or more data packets, transmits back to the server PC that the one or more data packets was received.

19. The system according to claim 17 wherein the one or more data packets do not have a confirmation receipt.

20. The system according to any preceding claim wherein the server PC has means for accepting a first handshake data packet from the client PC.

21. The system according to claim 20 wherein the first handshake data packet from the client PC instructs the server PC to only accept data from the IP address of the client PC.

22. The system according to any preceding claim wherein the server PC has means for limiting the acceptance of instructions to the server PC by the client PC at a first IP address.

23. The system according to any preceding claim wherein the server PC has means for communicating to the medical device through an electromagnetic coupling

24. The system according to claim 23 wherein the electromagnetic coupling comprises a head transmitting and receiving electromagnetic signals to the medical device

25. The system according to any preceding claim wherein the server PC has means for transmitting an applet to the client.

26. The system according to claim 25 wherein the applet comprises an executable program which performs tasks on the client PC without having to send a request back to the server PC.

27. The system according to any preceding claim wherein the medical device has means for generating Java applets.

28. The system according to any preceding claim wherein the server PC has means for determining the delay between data sent from the server PC to its receipt by the client PC, the client PC having means for displaying to a user the determined delay at pre-selected times.

29. The system according preceding claim 2, wherein the programmer is configured to receive data from the medical device, the system comprising:
a means for dis-assembling the received data into formatted packets of data for transmission through the dispersed communication pathway; and
means for transmitting the formatted packets through the dispersed communication pathway.

30. The system according to claim 29 further comprising a means for re -assembling formatted packets of data into re-produced data, the re-produced data corresponding to the received data.

31. The system according to claim 30 further comprising means to display the re-produced data wherein said display means has inputs to input commands to the implanted device.

## Patentansprüche

1. System zur Fernkommunikation mit einer medizinischen Vorrichtung mit:
einer medizinischen Vorrichtung, die dafür eingerichtet ist, in einen Patienten implantiert zu werden;
einem Server-PC (221), der mit der medizinischen Vorrichtung kommuniziert, wobei der Server-PC (SPC) Mittel aufweist, um Daten über einen verteilten Datenkommunikationspfad zu übertragen; und
einem Client-PC (225), der Mittel aufweist, um Daten zu empfangen, die über einen verteilten Kommunikationspfad von dem Server-PC übertragen werden,
wobei der Server-PC Mittel zum Übertragen von Daten über einen verteilten Datenkommunikationspfad entlang einem ersten Kanal (10) und einem zweiten Kanal (11) aufweist,
wobei der Client-PC Mittel zum Empfangen von Daten, die über einen verteilten Kommunikationspfad von dem Server-PC entlang dem ersten Kanal und dem zweiten Kanal übertragen werden, aufweist, **dadurch gekennzeichnet, dass** der erste Kanal (10) ein erstes Datenprotokoll verwendet, das eine Empfangsbestätigung erfordert, die entlang dem ersten Kanal zurück zu übertragen ist, und der zweite Kanal (11) ein zweites Datenprotokoll verwendet, dass keine Empfangsbestätigung erfordert, und das eine Übertragung von Daten nur in einer Richtung bereitstellt.

2. System nach Anspruch 1, ferner mit einer Programmiereinrichtung (2), wobei die Programmiereinrichtung mit dem Server-PC verbunden ist und Mittel zum Interagieren mit einer medizinischen Vorrichtung bereitstellt.

3. System nach Anspruch 2, bei dem die Mittel zum Interagieren mit einer medizinischen Vorrichtung Mittel zum Testen der medizinischen Vorrichtung aufweisen.

4. System nach Anspruch 2 oder 3, bei dem die Programmiereinrichtung (4) Mittel zum Erfassen einer Verbindungsunterbrechung zwischen dem Client-PC und dem Server-PC oder dem Server-PC und der Programmiereinrichtung aufweisen.

5. System nach Anspruch 4, bei dem die Programmiereinrichtung (2) Mittel zum Beenden jedes durch die medizinische Vorrichtung durchgeführten Tests aufweist, sollten die Mittel zum Erfassen der Verbindungsunterbrechung eine Verbindungsunterbrechung erfassen.

6. System nach Anspruch 2, bei dem die Mittel zum Interagieren mit einer medizinischen Vorrichtung Mittel zum Abfragen der medizinischen Vorrichtung aufweisen.

7. System nach Anspruch 2, bei dem die Mittel zum Interagieren mit einer medizinischen Vorrichtung Mittel zum Downloaden von Daten von der medizinischen Vorrichtung aufweisen.

8. System nach Anspruch 2, bei dem die Mittel zum Interagieren mit einer medizinischen Vorrichtung Mittel zum Uploaden von Daten an die medizinische Vorrichtung aufweisen.

9. System nach einem der vorstehenden Ansprüche, bei dem der Client-PC Mittel zum Kommunizieren über einen verteilten Datenkommunikationspfad an den Server-PC aufweist.

10. System nach einem der vorstehenden Ansprüche, bei dem der Client-PC Mittel zum Klassifizieren von Client-Benutzern als entweder erste Client-Benutzer oder zweite Client-Benutzer aufweist.

11. System nach einem der Ansprüche 1 bis 9, bei dem der Client-PC Mittel zum Klassifizieren von Client-Benutzern als entweder erste Client-Benutzer oder zweite Client-Benutzer oder dritte Client-Benutzer oder vierte Client-Benutzer aufweist.

12. System nach Anspruch 10 oder 11, das ferner Mittel zum Bereitstellen eines ersten Benutzerinterfaces an den ersten Client-Benutzer auf die Klassifizierung des Client-Benutzers als erster Client-Benutzer hin aufweist.

13. System nach Anspruch 12, das ferner Mittel zum Bereitstellen eines zweiten Benutzerinterfaces an den Client-Benutzer auf die Klassifizierung des Client-Benutzers als zweiter Client-Benutzer hin aufweist.

14. System nach einem der Ansprüche 10, 11, 12, oder 13, das ferner Mittel zum Beschränken eines Zugangs zu der medizinischen Vorrichtung auf die Klassifizierung des Client-Benutzers als erster Client-Benutzer hin aufweist.

15. System nach Anspruch 14, bei dem die Mittel zum Beschränken des Zugangs zu der medizinischen Vorrichtung auf das Klassifizieren des Client-Benutzers als erster Client-Benutzer hin Mittel aufweist, um dem ersten Client-Benutzer lediglich das Beobachten des Betriebes der medizinischen Vorrichtung zu gestatten.

16. System nach Anspruch 15, bei dem die medizinische Vorrichtung Mittel zum Speichern von Informationen aufweist, wobei die Mittel zum Beschränken des Zugangs zu der medizinischen Vorrichtung auf das Klassifizieren des Client-Benutzers als erster Client-Benutzer hin Mittel aufweist, um dem ersten Client-Benutzer zu gestatten, lediglich Informationen, die in der medizinischen Vorrichtung gespeichert sind, zu empfangen.

17. System nach einem der vorstehenden Ansprüche, bei dem die Mittel zum Übertragen von Daten über einen verteilten Datenkommunikationspfad Mittel zur Übertragung von Daten in einem oder mehreren Datenpaketen aufweisen.

18. System nach Anspruch 17, bei der das eine oder die mehreren Datenpakete jeweils eine Bestätigungsquittung aufweisen, wodurch der Client-PC, auf den Empfang eines jeden des einen oder der mehreren Datenpakete hin, an den Sever-PC zurück überträgt, dass das eine oder die mehreren Datenpakete empfangen wurde bzw. wurden.

19. System nach Anspruch 17, bei dem das eine oder die mehreren Datenpakete keine Bestätigungsquittung aufweisen.

20. System nach einem der vorstehenden Ansprüche, bei dem der Server-PC die Mittel zum Akzeptieren bzw. zur Annahme eines ersten Handshake-Datenpakets von dem Client-PC aufweist.

21. System nach Anspruch 20, bei dem das erste Handshake-Datenpaket von dem Client-PC den Server-PC anweist, nur Daten von der IP-Adresse des Client-PC zu akzeptieren.

22. System nach einem der vorstehenden Ansprüche, bei dem der Server-PC Mittel zum Beschränken der Akzeptanz bzw. Annahme von Anweisungen an den Server-PC durch den Client-PC bei einer ersten IP-Adresse aufweist.

23. System nach einem der vorstehenden Ansprüche, bei dem der Server-PC Mittel zum Kommunizieren mit der medizinischen Vorrichtung über eine elektromagnetische Kopplung aufweist.

24. System nach Anspruch 23, bei dem die elektromagnetische Kopplung einen Kopf, der elektromagnetischen Signale an die medizinische Vorrichtung überträgt und sie von ihr empfängt, aufweist.

25. System nach einem der vorstehenden Ansprüche, bei dem der Server-PC Mittel zum Übertragen eines Applets an den Client aufweist.

26. System nach Anspruch 25, bei dem das Applet ein ausführbares Programm aufweist, welches Aufgaben auf dem Client-PC ausführt, ohne eine Anfrage zurück an den Server-PC senden zu müssen.

27. System nach einem der vorstehenden Ansprüche, bei dem die medizinische Vorrichtung Mittel zum Erzeugen von Java-Applets aufweist.

28. System nach einem der vorstehenden Ansprüche, bei dem der Server-PC Mittel zum Bestimmen der Verzögerung zwischen von dem Server-PC gesendeten Daten bis zu ihrem Empfang durch den Client-PC aufweist, wobei der Client-PC Mittel zum Anzeigen der bestimmten Verzögerung zu vorausgewählten Zeiten an den Benutzer aufweist.

29. System nach Anspruch 2, bei dem die Programmiereinrichtung dazu eingerichtet ist, Daten von der medizinischen Vorrichtung zu empfangen, wobei das System aufweist:
Mittel zum Disassemblieren der empfangenen Daten in formatierte Pakete von Daten zur Übertragung zwischen bzw. über den verteilten Kommunikationspfad.

30. System nach Anspruch 29, ferner mit Mitteln zum erneuten Assemblieren von formatierten Datenpaketen in reproduzierte Daten, wobei die reproduzierten Daten den empfangenen Daten entsprechen.

31. System nach Anspruch 30, ferner mit Mitteln zum Anzeigen der reproduzierten Daten, wobei die Anzeigemittel Eingänge zur Eingabe von Befehlen an die implantierbare Vorrichtung aufweisen.

## Revendications

1. Système pour une communication à distance avec un dispositif médical implantable comportant :
un dispositif médical adapté pour être implanté dans un patient ;
un PC serveur (221) communiquant avec le dispositif médical, le PC serveur (SPC) et ayant des moyens pour transmettre des données à travers un trajet de communication de données dispersé ; et
un PC client (225) ayant des moyens pour recevoir des données transmises à travers un trajet de communication dispersé en provenance du PC serveur,
le PC serveur ayant des moyens pour transmettre des données à travers un trajet de communication de données dispersé le long d'un premier canal (10) et d'un second canal (11),
le PC client ayant des moyens pour recevoir des données transmises à travers un trajet de communication dispersé en provenance du PC serveur le long dudit premier canal et dudit second canal,
**caractérisé en ce que** le premier canal (10) utilise un premier protocole de données nécessitant une confirmation de réception à retransmettre le long dudit premier canal, et le second canal (11) utilise un second protocole de données ne nécessitant pas de confirmation de réception et permettant une transmission de données seulement dans une direction.

2. Système selon la revendication 1, comportant en outre un programmeur (2), le programmeur étant couplé au PC serveur et fournissant des moyens pour interagir avec un dispositif médical.

3. Système selon la revendication 2, dans lequel les moyens pour interagir avec un dispositif médical comportent des moyens pour tester le dispositif médical.

4. Système selon la revendication 2 ou 3, dans lequel le programmeur (4) a des moyens pour détecter une interruption de connexion entre le PC client et le PC serveur ou le PC serveur et le programmeur.

5. Système selon la revendication 4, dans lequel le programmeur (2) a des moyens pour terminer un test quelconque étant exécuté par le dispositif médical si les moyens pour détecter l'interruption de connexion détectent une interruption de connexion.

6. Système selon la revendication 2, dans lequel les moyens pour interagir avec un dispositif médical comportent des moyens pour interroger le dispositif médical.

7. Système selon la revendication 2, dans lequel les moyens pour interagir avec un dispositif médical comportent des moyens pour télécharger des données à partir du dispositif médical.

8. Système selon la revendication 2, dans lequel les moyens pour interagir avec un dispositif médical comportent des moyens pour télécharger des données vers le dispositif médical.

9. Système selon l'une quelconque des revendications précédentes, dans lequel le PC client a des moyens pour communiquer à travers un trajet de communication de données dispersé avec le PC serveur.

10. Système selon l'une quelconque des revendications précédentes, dans lequel le PC client a des moyens pour classer des utilisateurs clients soit en tant que premier utilisateur client soit en tant que deuxième utilisateur client.

11. Système selon l'une quelconque des revendications 1 à 9, dans lequel le PC client a des moyens pour classer des utilisateurs clients soit en tant que premier utilisateur client, soit en tant que deuxième utilisateur client, soit en tant que troisième utilisateur client, soit en tant que quatrième utilisateur client.

12. Système selon la revendication 10 ou 11, comportant en outre des moyens pour fournir une première interface utilisateur au premier utilisateur client lors de la classification de l'utilisateur client en tant que premier utilisateur client.

13. Système selon la revendication 12, comportant en outre des moyens pour fournir une deuxième interface utilisateur à l'utilisateur client lors de la classification de l'utilisateur client en tant que deuxième utilisateur client.

14. Système selon la revendication 10, 11, 12 ou 13, comportant en outre des moyens pour limiter l'accès au dispositif médical lors de la classification de l'utilisateur client en tant que premier utilisateur client.

15. Système selon la revendication 14, dans lequel les moyens pour limiter l'accès au dispositif médical lors de la classification de l'utilisateur client en tant que premier utilisateur client comportent des moyens pour permettre au premier utilisateur client d'observer uniquement le fonctionnement du dispositif médical.

16. Système selon la revendication 15, dans lequel le dispositif médical a des moyens pour mémoriser des informations, et dans lequel les moyens pour limiter l'accès au dispositif médical lors de la classification de l'utilisateur client en tant que premier utilisateur client comportent des moyens pour permettre au premier utilisateur client de récupérer uniquement des informations mémorisées dans le dispositif médical.

17. Système selon l'une quelconque des revendications précédentes, dans lequel les moyens pour transmettre des données à travers un trajet de communication de données dispersé comportent des moyens pour transmettre des données dans un ou plusieurs paquets de données.

18. Système selon la revendication 17, dans lequel le paquet ou les paquets de données ont chacun une réception de confirmation de sorte que le PC client, lors de la réception du paquet de données ou de chacun des paquets de données, retransmet au PC serveur que le paquet ou les paquets de données ont été reçus.

19. Système selon la revendication 17, dans lequel le paquet ou les paquets de données n'ont pas de réception de confirmation.

20. Système selon l'une quelconque des revendications précédentes, dans lequel le PC serveur a des moyens pour accepter un premier paquet de données d'établissement de liaison (handshake) en provenance du PC client.

21. Système selon la revendication 20, dans lequel le premier paquet de données d'établissement de liaison en provenance du PC client ordonne au PC serveur d'accepter uniquement des données en provenance de l'adresse IP du PC client.

22. Système selon l'une quelconque des revendications précédentes, dans lequel le PC serveur a des moyens pour limiter l'acceptation d'instructions dans le PC serveur par le PC client à une première adresse IP.

23. Système selon l'une quelconque des revendications précédentes, dans lequel le PC serveur a des moyens pour communiquer avec le dispositif médical via un couplage électromagnétique.

24. Système selon la revendication 23, dans lequel le couplage électromagnétique comporte une tête transmettant des signaux électromagnétiques au dispositif médical et recevant des signaux électromagnétiques en provenance de celui-ci.

25. Système selon l'une quelconque des revendications précédentes, dans lequel le PC serveur a des moyens pour transmettre un applet au PC client.

26. Système selon la revendication 25, dans lequel l'applet comporte un programme exécutable qui exécute des tâches sur le PC client sans avoir à renvoyer une demande au PC serveur.

27. Système selon l'une quelconque des revendications précédentes, dans lequel le dispositif médical a des moyens pour générer des applets Java.

28. Système selon l'une quelconque des revendications précédentes, dans lequel le PC serveur a des moyens pour déterminer le délai entre des données envoyées depuis le PC serveur jusqu'à leur réception par le PC client, le PC client ayant des moyens pour afficher à un utilisateur le délai déterminé selon à des moments présélectionnés.

29. Système selon la revendication précédente 2, dans lequel le programmeur est configuré pour recevoir des données en provenance du dispositif médical, le système comportant :
des moyens pour désassembler les données reçues en paquets de données formatés en vue de leur transmission à travers le trajet de communication dispersé ; et
des moyens pour transmettre les paquets formatés à travers le trajet de communications dispersé.

30. Système selon la revendication 29, comportant en outre des moyens pour réassembler des paquets de données formatés en données reproduites, les données reproduites correspondant aux données reçues.

31. Système selon la revendication 30, comportant en outre des moyens pour afficher les données reproduites, lesdits moyens d'affichage ayant des entrées pour entrer des instructions dans le dispositif implanté.
